# EUROPEAN PATENT APPLICATION

(11) **EP 2 742 961 A1**
(43) Date of publication of application: **18.06.2014**
(21) Application number: 12196882.0
(22) Date of filing: 13.12.2012
(51) Int. Cl.: A61M 5/14, G06F 19/00

(54) **Modular drug infusion system with central control and multiple infusion pumps comprising touch screens**

(71) Applicant: Palletti, Stephen, London E18 1EP (GB)
(72) Inventor: Palletti, Stephen, London E18 1EP (GB)

(57) **Abstract**

A modular drug infusion system (200) includes a plurality of infusion pump modules (230-260) and a central management unit (210) interconnected with the infusion pump modules to provide centralized control. The central management unit includes a display and touch screen (211) that enables users to initiate infusion related parameters and calculation for each of the pump modules. The pump modules also include display and touch screens (211) that enable the user to initiate infusion related parameters and calculations in each pump without having to access the central management unit.

## Description

### BACKGROUND OF THE INVENTION

### Technical Field

This invention relates generally to drug delivery systems, and more particularly to a centrally managed system with multiple infusion pumps that provide a patient with metered or measured doses of various intravenous medications.

### Background information

A centrally managed, multi-pump, drug infusion system alleviates many concerns in such environments as intensive care units, cardiac units, operating rooms, and trauma centers. Capable of infusing several drugs simultaneously and doing so at different points of the body in a manner accommodating incompatible drugs, the system provides an organized complement of components suited for use under crisis conditions.

One such system includes a microprocessor -based central management unit mounted together with infusion pump modules on an IV stand. The central management unit controls the internal setup of the pumps to cause them to function as desired. In addition, it receives and displays information from the pump modules on a touch screen that also serves as an input device with which the user can enter data and commands to the central management unit.

The central management unit typically displays a menu of various commands or operating parameters, and the user simply point at the command or parameter selected. Operation typically proceeds in one or two ways. In what may be called dosage-based operations, the physician specifies a dosage to be administered, with operation involving calculation of the corresponding infusion rate so that the infusion pump module can be controlled accordingly. In rate-based operation, however, there is no need to calculate the rate because it is specified. Instead in rate-based operation involves calculation of the corresponding dosage, this being done so that a record may be kept of the dosage that is administered.

In either case, a calculation or other medication delivery parameters must be made or delivered, and it is therefore desirable that the infusion system include some mechanism for doing this in a manner suited to crisis conditions and medication administration. The calculation and/or medication administration process should be quick with little room for user confusion and error.

Although calculators and other infusion parameter controllers exist, they are not configured for this special function. With some calculators the specified dosage or infusion parameters must be manually entered with the necessary values.

Furthermore, data for one infusion pump module is often lost when the calculator is used for another infusion pump module, and this compounds the problem. In addition, it is sometimes desirable to perform dose and infusion rate calculation during titration procedures and existing calculator arrangement are inconvenient to use for this purpose. Some prior art solutions include systems that provide touch screen technology with calculator arrangements that automatically calculates and displays and stores a separate set of parameter values for each of a plurality of infusion modules in a central management unit.

However, with modem hospitals having developed central systems using sophisticated computer networking equipment to help track patients and monitor dosage calculations and infusion parameter values, the prior calculator solution having several short-comings including the lack of wireless connections to the hospital information systems and the tying of the calculation and infusion parameter values to the central management unit.

It is therefore desirable to have a infusion system having a plurality of infusion modules with smart connection technology and the versatility to perform functions normally delegated to a central infusion management unit. It is also desirable to have an infusion system with capability to wirelessly communicate information between the infusion central management unit and the infusion modules coupled the central management unit using modem communication channels such as NFC.

### SUMMARY OF THE INVENTION

The invention solves the problems outlined above by providing a infusion system having a central infusion management unit with multiple infusion modules each of the central infusion management unit and the infusion modules having touch screen calculator arrangement that automatically calculates, displays and stores separate set of infusion parameters centrally in the central management unit and separately in each infusion module. The invention also provides a wireless communication system whereby infusion parameter information in the individual infusion modules could be proximally communicated to the central management unit. The invention further provides for power management mechanism to reduce and management power supply to the central management unit and individual infusion pumps in a way to save and reduce power consumption.

According to the present invention there is provided a drug infusion system comprising:
a central infusion control device having a central touch screen for providing user interface to a plurality of infusion pumps coupled to the central infusion control device:
   a plurality of infusion pump modules coupled to the central infusion control device each of the plurality of pump modules having touch screens for providing user interface to infusion related information to operate the plurality of infusion modules, and wherein the touch screen display provides an infusion menu including a calculator screen to be used for calculating infusion related parameters;
   storing means in the central infusion control device or storing separate infusion related parameters for each of the plurality of infusion pump modules; and
   storing means in each of the plurality of infusion pump modules for storing infusion related parameters in each of the plurality of infusion pump modules.

Preferably, the touch screen display in either the central infusion pump or each of the plurality of infusion pump module include a titrate screen for titrating purposes.

Conveniently, the plurality of infusion pump modules includes wireless control units to enable each one of the plurality of the infusion pump modules to wirelessly connect to the central infusion control device.

Advantageously, each one of the plurality of infusion pump modules has a power management unit to manage power consumption in the infusion pump module.

The present invention also provides a drug infusion system, comprising: a central infusion management unit; a plurality of infusion pumps coupled to the central infusion management unit; a IV pole including a power inductor for providing power to the plurality of the plurality of infusion pumps and the central infusion management unit, wherein the central infusion management unit and the plurality of infusion pumps inductively couple to the IV pole.

Preferably, the drug infusion system further comprises infusion parameter calculators in each of the plurality of infusion pumps and the central management unit for calculating infusion rates or drugs infused from the plurality of infusion pumps and the central infusion management unit.

Conveniently, each of the plurality of infusion pumps and the central in fusion management unit has a touch screen to provide user interface to the calculators for calculating infusion-rated parameters.

Advantageously, each of the plurality of infusion pumps includes a power inductor for inductively powering the pump.

Conveniently, each one of the plurality of infusion pumps also includes a battery for powering the power when the pump is disconnected from the IV pole.

Preferably, each of the plurality of the infusion pumps includes a power management unit for managing power consumption in the infusion pump module.

Advantageously, each of the plurality of the infusion pumps includes a wireless management unit which enables each of the plurality of infusion pumps to wirelessly communicate with the central infusion management unit.

Preferably, the wireless communication protocol is a Near Field Communication.

Advantageously, the wireless communication protocol is a WiFi based communication.

### BRIEF DESCRIPTION OF THE DRAWINGS

The foregoing objects, advantages and features, as well as other objects and advantages, will become more apparent with reference to the description and drawings below, in which like numeral represent like elements and in which:
FIG. 1 is a prior art schematic depiction of a frontal elevation view of a IV stand supporting a drug infusion system with a central infusion management unit with touch screen technology to store calculator and other infusion parameters for each one of the infusion modules in the system;
FIG. 2 is a schematic depiction of a frontal elevation view of an IV stand supporting a drug infusion system constructed according to the invention;
FIG. 3 is schematic block diagram of the system connected to a hospital information system;
FIG. 4 is a schematic block diagram depiction of the touch screen circuitry of the system;
FIG. 5 is a schematic block diagram depiction of the infusion pumping device according to the invention;
FIG. 6A is a schematic block diagram of the system of the invention showing the power supply mechanism;
FIG. 6B is a schematic block diagram of the system showing the communication mechanism; and
FIGS. 7 depict various infusion parameter displays utilized by the infusion pumping devices and the central infusion management device according to the invention.

### DETAILED DESCRIPTION

FIG_{.} 1 shows an infusion system 100 of the prior art that is mounted to IV pole 110. The system 100 includes a central infusion management device 120 that is interconnected with a plurality of infusion pumps modules 130,140,150 and 160, the infusion pump modules providing a patient with metered doses of various intravenous medications under centralized control of the central management device 120. Fluid flows from a plurality of IV bottles and through tubes to the infusion pump modules for this purpose with the central management device 120 controlling such aspects of operation as an infusion rate for each of the modules.

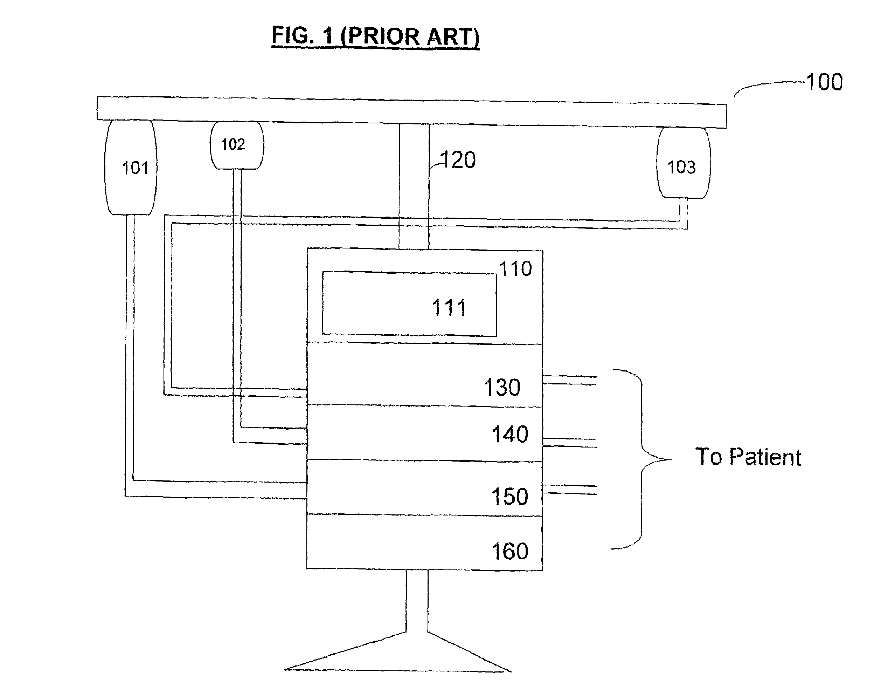

The central management device 120 includes a touch screen 125 and associated components that serve as a user interface to communicate with the central management device 120. In the system shown in Fig. 1, a user is able to initiate a menu of infusion related parameters for each of the infusion modules. Each of the infusion related information, such as dose rate calculations pertaining to each pump module is stored in the central management device 120.

The touch screen calculator arrangement in the central management device 120 automatically calculates, displays and stores separate set of parameter values for each the infusion pump modules 130 -160. The system 100 shown in FIG.1 does not have the capability of each of the infusion pump module 130 -160 having the ability to individually provide a touch screen that allow the automatic calculators and other patient specific medication delivery parameters to be displayed and stored in the individual pump.

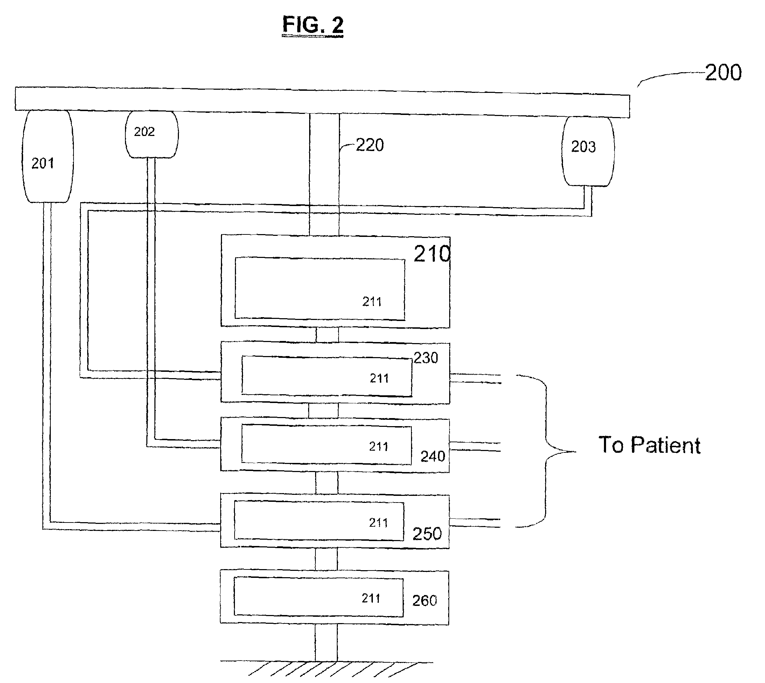

FIG.2 shows an infusion system 200 of the prior art that is mounted to IV pole 210. The system 100 includes a central infusion management device 220 that is interconnected with a plurality of infusion pumps modules 230,240,250 and 260, the infusion pump modules providing a patient with metered doses of various intravenous medications under centralized control of the central management device 220. Fluid flows from a plurality of IV bottles and through tubes to the infusion pump modules for this purpose with the central management device 220 controlling such aspects of operation as an infusion rate for each of the modules.

The central management device 220 includes a touch screen 225 and associated components that serve as a user interface to communicate with the central management device 220. In the system shown in Fig. 2, a user is able to initiate a menu of infusion related parameters for each of the infusion modules. Each of the infusion related information, such as dose rate calculations pertaining to each pump module is stored in the central management device 220.

The touch screen calculator arrangement in the central management device 220 automatically calculates, displays and stores separate set of parameter values for each the infusion pump modules 230 -260. In the system showed in FIG. 2 each of the infusion pumps 230 - 260 has touch screen displays that allows a user to be able to initiate a menu of infusion related parameter for the pump. Each pump is also able to locally store the infusion parameters.

In one embodiment, the infusion pump modules 230 -260 has wireless unit with each pump 230 -260 provided with a unique pump ID such as an identification code, or a wireless signal identifier. Each of the pumps 230 -260 can wirelessly communicate with a hospital information system and the information regarding a particular pump 230 - 260 can be stored in the hospital information system or in the central management unit 210. The wireless unit in each of the pumps 230 - 260 also allows information exchange between the central management unit 210 and a pump 230 - 260 if the pump is disconnected from the IV pole 220.

For purposes of operating the pump devices 230 - 260 according to one embodiment of the present invention, a power management unit is provided in each pump 230 -260 which enables the pump to inductively connect to the IV pole 220 without the need for directly plugging a pump 230 -260 into a power socket. The infusion pumping devices230 -260 are also provided with batteries to enable each pump to be disconnected from the IV pole 220 and still operate. In another embodiment the power management unit also provides in each of the pumping devices 230 -260 to automatically go into standby mode when a device is not in active use.

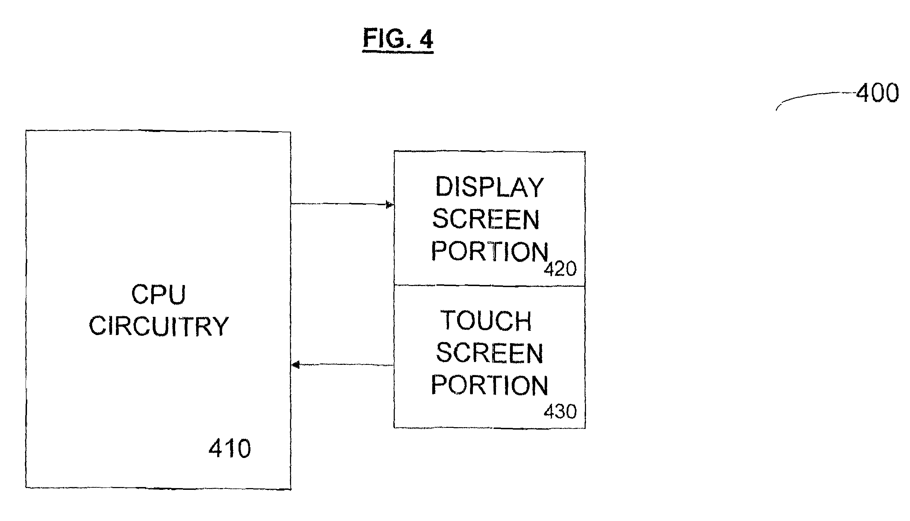

The touch screen technology adopted in each of the infusion pumps 230 -260 in the present invention is deployed in both the central management unit and the individual infusion devices. The touch screen and associated components are illustrated in FIG. 4. As showed in FIG.4 the touch screen part includes a central processing unit (CPU) 410 and ports to which the individual pumps connect to the central infusion management unit 210. The CPU circuitry 410 may include a conventional microprocessor with suitable programming, memory and supporting circuitry that are not individually shown.

Control information is communicated between the CPU circuitry 410 and the infusion pump devices using the circuitry information to display information on the display screen portion 420 of the touch screen and receive information from the touch input portion 430. Each of the pump devices is capable of displaying relevant infusion parameter information.

The invention includes a mechanism for enabling a user to initiate the display menu of infusion related parameters on any of the individual infusion pumps 230 - 260 or the central infusion management unit 210. The primary entry display on the central infusion management unit 210 may be utilized to designate a selected i one of the individual infusion pumps 230 - 260 to either display infusion parameter information or to configure such parameter information.

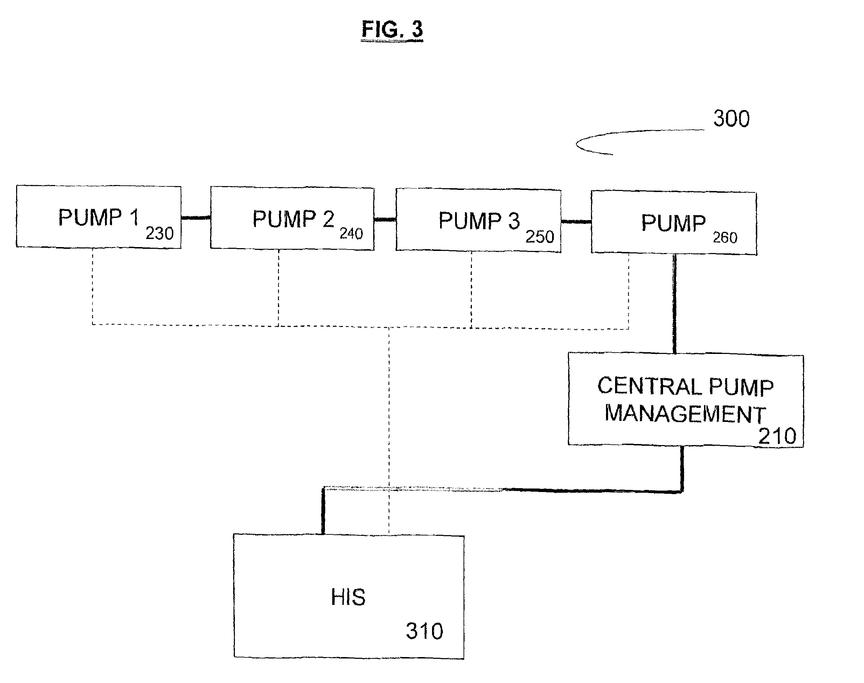

FIG. 3 is a schematic block diagram depicting one embodiment of the invention connected to a hospital information system (HIS) 310. In the example shown in FIG. 3, the central infusion pump control unit 210 is coupled to the HIS 310 to receive and transmit drug infusion information including the infusion rate calculator parameters for display in the control unit 210. Also each of the pumps 230 -260 are capable of receiving infusion related parameters from the HIS 310.

FIG. 4 is a schematic block diagram of the touch screen circuitry of the invention. The system showed in FIG. 4 includes CPU circuitry 410 coupled to display screen 420 and touch screen portion 430. In operation, control information pertaining to infusion related parameters is transmitted to the display screen 420. Communication between user initiated commands, menus, etc., is transmitted from the touch screen portion to the CPU circuitry 410.

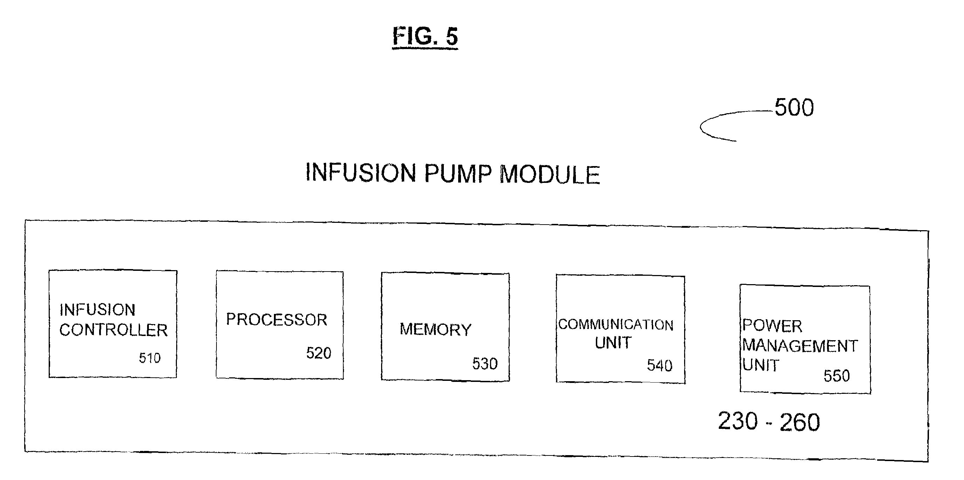

FIG. 5 is a schematic block diagram depiction of one embodiment of the pump devices 230 - 260 of the invention. The pump device includes an infusion controller 510 for controlling infusion parameter and management in each of the pump devices, a processor 520, memory 530, communication unit 540 and power unit 550.

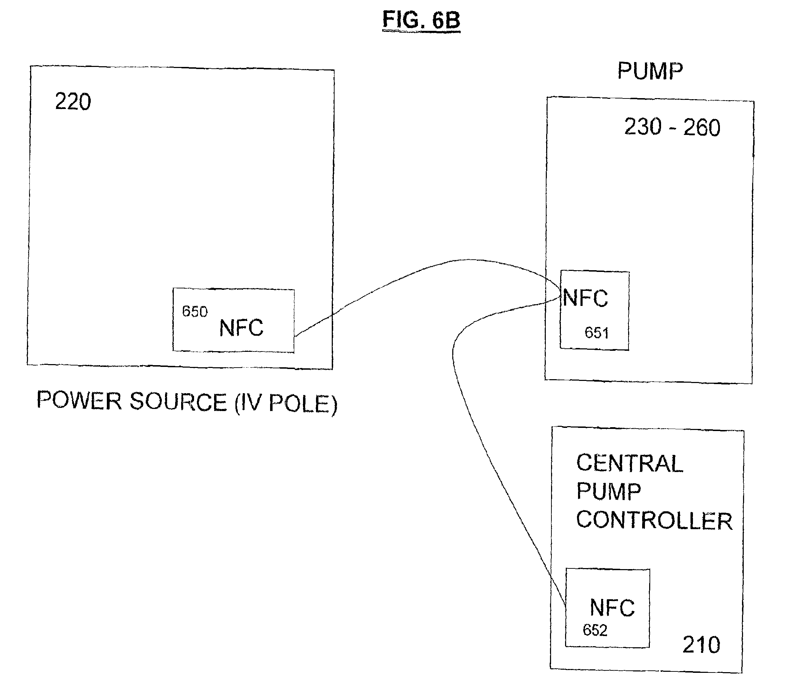

In one embodiment of the invention communication unit 540 enables the pump devices 230 - 260 to wireless connect to the central pump management unit 210 to transmit and receive infusion parameter information as well as patient specific information. In one embodiment, the communication unit 540 may employ a Near Field Communication technology to establish radio communication (FIG.6B) with either the central infusion management unit 210 or each individual infusion device 239 - 260.

Such Near Field Communication may be achieved by either unplugging an individual infusion device operating on battery and bringing it in close proximity to the central infusion management unit to transmit data. The individual infusion pump device may also communicate via the communication unit when connected to the IV pole 220.

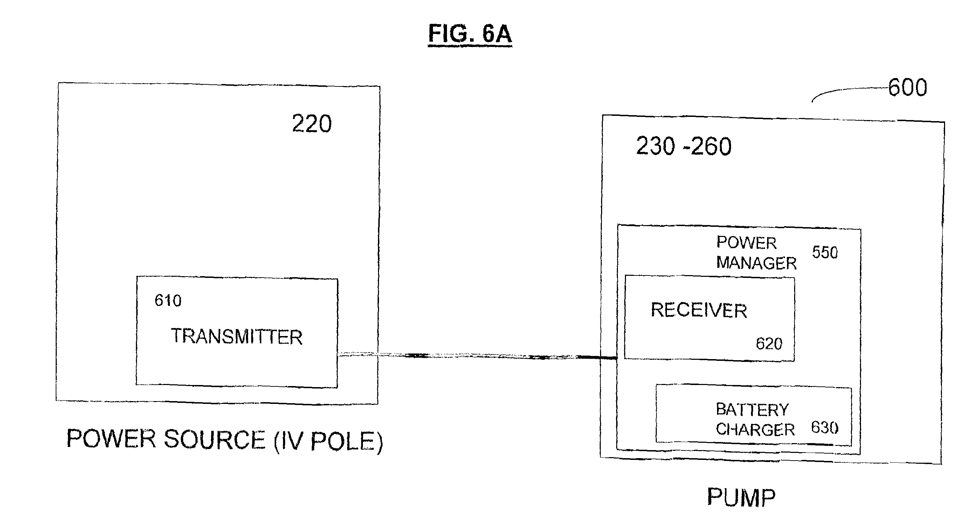

The power management unit 550 enables the pump devices to inductively connect to a power source such as the IV pole 220 which is always plugged into a power source. Inductive coupling consists of a system of coils (FIG. 6A), a transmitter coil in the IV pole 220 and a receiver coil in the infusion pump 230 - 260. Both coils form a system of magnetically coupled inductors. An alternate current in the transmitter coil 610 generates a magnetic field which induces a voltage in the receiver coil 620.

The voltage can not only be used to power pump units but also charge the batteries in the pumps. In one embodiment, the power management unit 550 manages power consumption in the pump to ensure that when the pump is inoperative, drainage on the battery and the inductively induced power is minimized. In one embodiment power may be conductively supplied to the pumps from the IV rack 220.

In one embodiment of the invention, the power source may be an IV rack 220 in which the pumps 230 - 260 are stacked. In one embodiment power to the pumps 230 -260 may be conductively provided from the IV pole 220 to the pumps 230 - 260.

The power management unit 550 may also use the inductively supplied from the IV pole 240 to charge batteries in the pump 230 -260 and the central pump management unit 220. The power management unit 550 may also automatically switch the pumps 230 - 240 into standby mode when a particular pump is not being used to help conserve power consumption. This is important since because each one of the pumps 230 -260 has a touch screen, power consumption is high.

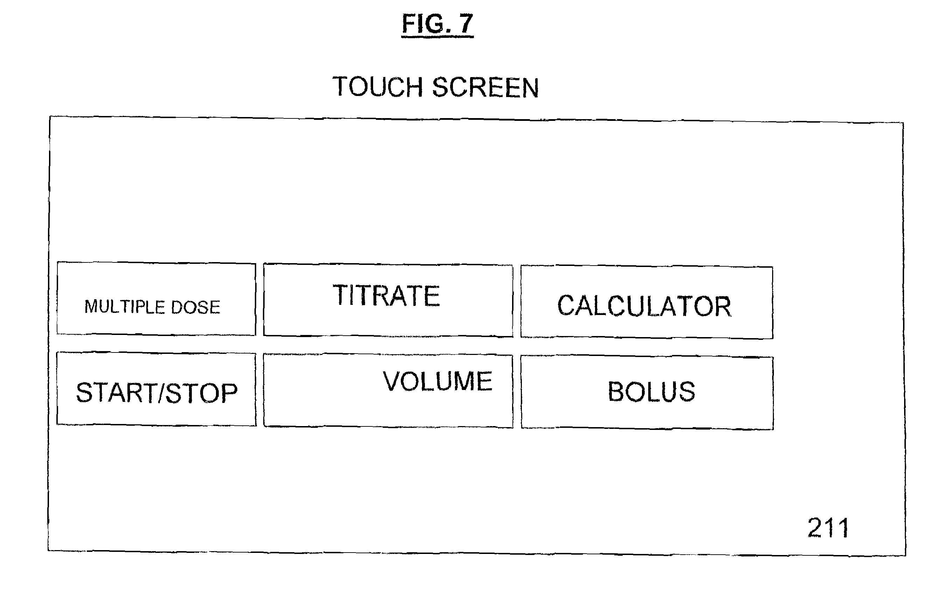

FIG. 7 shows one embodiment of the infusion parameter initiation display that enables a user to initiate a menu of infusion-rate related parameters of the invention. As showed in FIG. 7 the main entry of the display in the central management unit 220 may be utilized to designate one or more of the infusion pumps 230 -260 to allow a user to initiate infusion parameters of on-going infusion in one of the pumps 230 - 260 or patient specific infusion parameters.

In one embodiment the display is used to select one pf the special functions identified on that display for a designated infusion pump 230 -260 where the display being used is the main display on the central management unit 220 or on each individual display on the infusion pumps 230 - 260.

The invention therefore provides a system with touch screen infusion parameter related features such as calculator arrangement that may be shown in a central display in a central infusion controller or on individual touch screen displays on individual infusion pumps coupled to the central infusion controller.

Although an exemplary embodiment of the invention has been described with several inventive features, many modifications, changes and substitution may be made by one having ordinary skill in the art without necessarily departing from the spirits of this invention.

## Claims

1. A drug infusion system comprising:
a central infusion control device having a central touch screen for providing user interface to a plurality of infusion pumps coupled to the central infusion control device:
a plurality of infusion pump modules coupled to the central infusion control device each of the plurality of pump modules having touch screens for providing user interface to infusion related information to operate the plurality of infusion modules, and wherein the touch screen display provides an infusion menu including a calculator screen to be used for calculating infusion related parameters;
storing means in the central infusion control device or storing separate infusion related parameters for each of the plurality of infusion pump modules; and
storing means in each of the plurality of infusion pump modules for storing infusion related parameters in each of the plurality of infusion pump modules.

2. The drug infusion system of claim 1, wherein the touch screen display in either the central infusion pump or each of the plurality of infusion pump module include a titrate screen for titrating purposes.

3. The drug infusion system of claim 1 or claim 2, wherein the plurality of infusion pump modules includes wireless control units to enable each one of the plurality of the infusion pump modules to wirelessly connect to the central infusion control device.

4. The drug infusion system of any preceding claim, wherein each one of the plurality of infusion pump modules has a power management unit to manage power consumption in the infusion pump module.

5. A drug infusion system, comprising: a central infusion management unit; a plurality of infusion pumps coupled to the central infusion management unit; a IV pole including a power inductor for providing power to the plurality of the plurality of infusion pumps and the central infusion management unit, wherein the central infusion management unit and the plurality of infusion pumps inductively couple to the IV pole.

6. The drug infusion system at claim 5 further comprising infusion parameter calculators in each of the plurality of infusion pumps and the central management unit for calculating infusion rates or drugs infused from the plurality of infusion pumps and the central infusion management unit.

7. The drug infusion system of claim 5 or claim 6, wherein each of the plurality of infusion pumps and the central in fusion management unit has a touch screen to provide user interface to the calculators for calculating infusion-rated parameters.

8. The drug infusion system of any of claims 5 to 7, wherein each of the plurality of infusion pumps includes a power inductor for inductively powering the pump.

9. The drug infusion system of any of claims 5 to 8, wherein each one of the plurality of infusion pumps also includes a battery for powering the power when the pump is disconnected from the IV pole.

10. The drug infusion system of any of claims 5 to 9, wherein each of the plurality of the infusion pumps includes a power management unit for managing power consumption in the infusion pump module.

11. The drug infusion system of any of claims 5 to 10, wherein each of the plurality of the infusion pumps includes a wireless management unit which enables each of the plurality of infusion pumps to wirelessly communicate with the central infusion management unit.

12. The drug infusion system of any of claim 5 to 11, wherein the wireless communication protocol is a Near Field Communication.

13. The drug infusion system of any of claim 5 to 11, wherein the wireless communication protocol is a WiFi based communication.
